# EUROPÄISCHE PATENTSCHRIFT

(11) **EP 2 247 711 B1**
(45) Veröffentlichungstag und Bekanntmachung des Hinweises auf die Patenterteilung: **10.04.2013**
(21) Anmeldenummer: 09713247.6
(22) Anmeldetag: 12.02.2009
(51) Int. Cl.: C12M 1/02

(54) **BIOREAKTOR**
BIOREACTOR
BIORÉACTEUR

(30) Priorität: 21.02.2008 DE 102008010427
(43) Veröffentlichungstag der Anmeldung: 10.11.2010
(73) Patentinhaber: Sartorius Stedim Biotech GmbH, 37079 Göttingen (DE)
(72) Erfinder: REIF, Oscar-Werner, 30173 Hannover (DE); VAN DEN BOOGAARD, Jürgen, 37127 Dransfeld (DE); GRELLER, Gerhard, 37085 Göttingen (DE); LUDWIG, Jens, 37127 Jühnde (DE)
(74) Vertreter: Schneider, Peter Christian
(86) Internationale Anmeldenummer: PCT/EP2009/000961
(87) Internationale Veröffentlichungsnummer: WO 2009/103450

(56) Entgegenhaltungen:
- EP-A- 1 156 242
- WO-A-2005/104706
- DE-A1- 10 319 821
- DE-A1- 19 808 280
- US-A- 5 509 667
- US-A1- 2006 280 028

## Beschreibung

Die Erfindung betrifft einen Bioreaktor mit einem Behälter, einem Wellengehäuse zur Durchführung einer Welle durch eine Wandung, einem im Innenraum des Behälters mit der Welle verbundenem Rührer, einem außerhalb des Behälters angeordneten, mit der Welle verbindbaren Antrieb, wobei die Welle in dem Wellengehäuse über mindestens ein Kugellager gelagert und durch mindestens eine Dichtung gegenüber dem Wellengehäuse abgedichtet ist.

Bioreaktoren mit einem Behälter mit einer flexiblen wandung werden insbesondere als Einweg-Bioreaktoren in der Pharmazie und Biotechnologie zunehmend häufig verwendet.

Aus der US 2006/0280028 A1 ist ein Bioreaktor mit einem eine flexible Wandung aufweisenden Behälter bekannt, durch dessen Wandung eine Welle zum Antrieb eines in dem Innenraum des Behälters angeordneten Rührers hindurchgeführt wird. Ein Wellengehäuse ist über einen Dichtungsflansch mit der flexiblen Wandung verbunden. Die Welle ist in dem Wellengehäuse über zwei Kugellager gelagert. Die Welle weist einen radial angeordneten flachen Dichtungsbund auf, der in einem Absatz des Wellengehäuses angeordnet ist. Die den Innenraum des Behälters abgewandte äußere, ringförmige Stirnfläche ist gegenüber einer Grundfläche des Absatzes durch eine Lippendichtung angedichtet. Die den Innenraum des Behälters zugewandte innere ringförmige Stirnfläche des Bundes wird gegenüber dem verrasteten Bodenteil des Wellengehäuses durch zwei koaxial zueinander angeordnete Lippendichtungen abgedichtet.

Nachteilig dabei ist, dass der Aufbau des Wellengehäuses in Verbindung mit den Dichtungen relativ aufwendig und kostenintensiv und zudem relativ voluminös ist.

Weiterhin ist aus der US 2005/0239199 A1 und der WO 2005/104706 A ein Bioreaktor mit einem eine flexible Wandung aufweisenden Behälter bekannt, in dem über ein mit der flexiblen Wandung verbundenes Wellengehäuse eine Welle durch die flexible Wandung in den Innenraum des Behälters drehbar geführt wird. In der innenraumseitigen Öffnung des Wellengehäuses wird die Welle dabei von einer an ihr angeordneten radial abstehenden, ringförmigen Scheibe geführt, die gegenüber einem Absatz des Wellengehäuses durch zwei koaxial angeordnete Lippendichtungen abgedichtet ist.

Auch diese Vorrichtung weist die oben genannten Nachteile auf.

Aus der EP 1 156 242 A2 ist ein Radialwellendichtring mit zwei Dichtlippen bekannt, bei dem die auf der Hydraulikseite liegende Dichtlippe durch eine Ringwendelfeder an die abzudichtende Welle gedrückt wird.

Nachteilig dabei ist, dass diese Dichtungen als in Hydraulikflüssigkeit laufende Dichtungen bzw. mindestens als geschmiert laufende Dichtungen, in denen der Zwischenraum zwischen den Dichtlippen als Schmiermitteldepot genutzt wird, ausgebildet sind.

Aufgabe der vorliegenden Erfindung ist es daher, einen Bioreaktor mit einem Behälter zur Verfügung zu stellen, bei dem die Wellendurchführung durch eine Wandung, die beispielsweise flexibel ausgebildet ist, bei kompakter Bauweise einfach und kostengünstig und trotzdem flüssigkeits- und gasdicht erfolgt.

Die Aufgabe wird in Verbindung mit dem Oberbegriff des Anspruches 1 dadurch gelöst, dass die Dichtung als ein Radialwellendichtring mit zwei in Längsrichtung der Welle hintereinander angeordneten, auf der Welle trocken laufbaren Dichtlippen ausgebildet ist, dass der Radialwellendichtring zum Innenraum des Behälters hin eine geschlossene Stirnfläche aufweist, die in radialer Richtung in eine als Schutzlippe ausgebildete Dichtlippe übergeht, und dass der Radialwellendichtring zum außenliegenden Antrieb hin eine Dichtlippe aufweist, die von einem Federring dichtend gegen die Wellenoberfläche gedrückt wird.

Überraschender Weise hat sich gezeigt, dass bei Verwendung einer Radialwellendichtung mit zwei Lippen, wie sie zur Abdichtung von Ringspalten gegenüber rotierenden Wellen zwischen einer unter Druck stehenden Hydraulikseite und einer Atmosphärenseite im Nasslauf bzw. unter Schmierung der Dichtlippe bekannt sind, im vorliegendem Fall auch bei trocken laufenden Dichtlippen eine ausreichende Gas- und Flüssigkeitsabdichtung ermöglichen. Durch den Trockenlauf kann auf den Reaktorinhalt kontaminierende bzw. mit dem Reaktorinhalt reagierende Schmierstoffe verzichtet werden. Durch die Verwendung des mindestens einen Radialwellendichtringes kann an der Welle auf einen scheibenförmigen speziellen Dichtbund verzichtet werden. Das Wellengehäuse mit der Welle kann somit einfacher und kostengünstiger sowie kompakter ausgebildet sein.

Die von dem Einsatz eines Radialwellendichtrings in der Hydraulik bekannte Schutzlippe wird nicht auf der Atmosphärenseite sondern auf der Druckseite eingesetzt, d.h. der Radialwellendichtring wird gegenüber seiner bekannten Einsatzweise um 180° gedreht eingesetzt und erzielt dennoch die erwünschte gas- und flüssigkeitsdichte Abdichtung. Die erste als Schutzlippe ausgebildete Dichtlippe hält Partikel aus dem Innenraum des Behälters ab und schützt die zweite Dichtlippe vor die Abdichtung beeinträchtigenden Partikeln.

Nach einer weiteren bevorzugten Ausführungsform der Erfindung ist in Längsrichtung der Welle eine der ersten Dichtung benachbarte zweite Dichtung angeordnet. Dadurch, dass die beiden Dichtungen nicht in radialer Richtung koaxial zueinander, sondern in axialer Richtung hintereinander angeordnet sind, bleibt die Kompaktheit des Wellengehäuses im Wesentlichen erhalten. Die Dichtwirkung der beiden hintereinander angeordneten Radialwellendichtringe wird dabei erhöht.

Das dem Rührer bzw. dem Innenraum des Behälters abgewandte freie Ende der Welle ist an einer Antriebswelle eines Motors des Antriebs ankoppelbar. Dabei kann das freie Ende der Welle über ein Spannfutter an die Antriebswelle angekoppelt werden.

Gemäß einer weiteren bevorzugten Ausführungsform der Erfindung ist die Welle in ihrer Längsrichtung über den Sitz des Kugellagers fixiert, wobei das Kugellager in einem Absatz des Wellengehäuses angeordnet ist und über einen in einer Ringnut des Wellengehäuses angeordneten Sprengring in seiner Lage gesichert ist.

Insbesondere bei Verwendung des Bioreaktors als Einmalbeutel ist es möglich, auch das Wellengehäuse einschließlich Kugellager, Welle und Wellendichtring aus Kunststoff auszubilden.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:

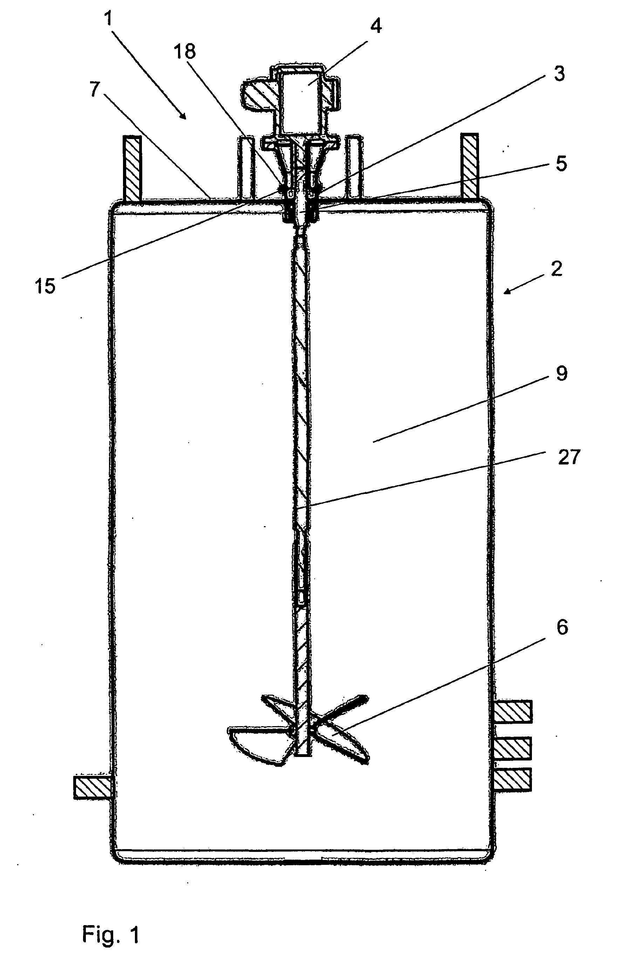

- Figur 1:: eine schematische Seitenansicht eines Bioreaktors,

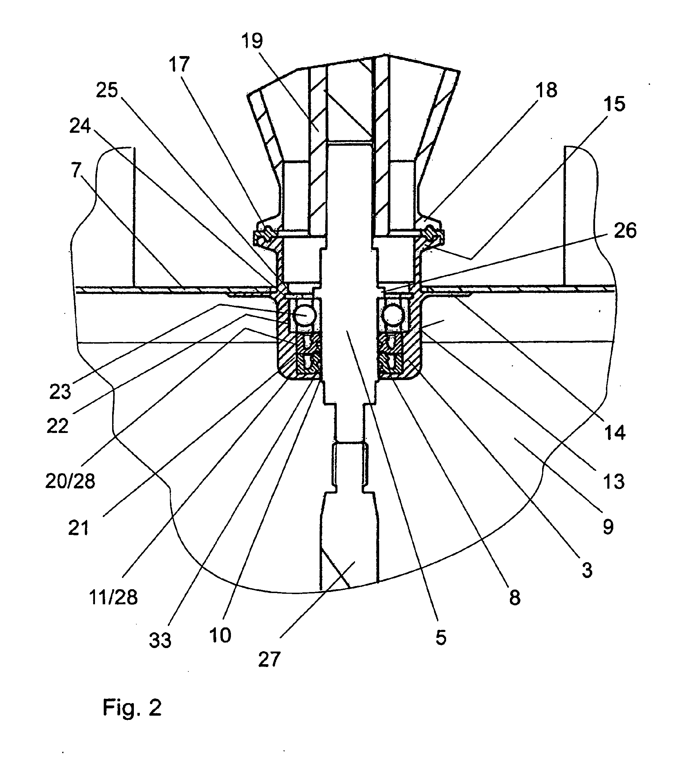

- Figur 2:: eine Seitenansicht des Wellengehäuses von Figur 1 in vergrößerter Darstellung im Ausriss,

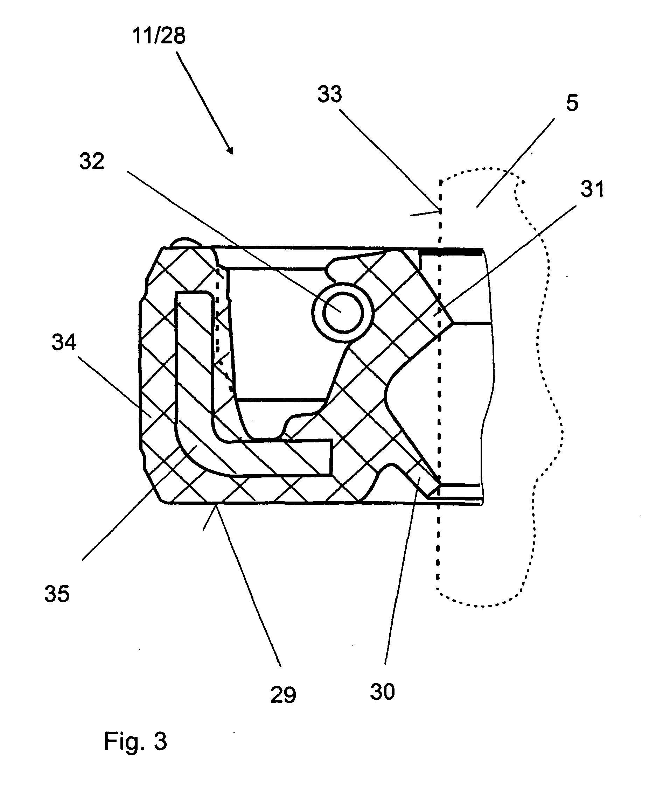

- Figur 3:: eine Seitenansicht im Schnitt und Ausriss eines Wellendichtringes und

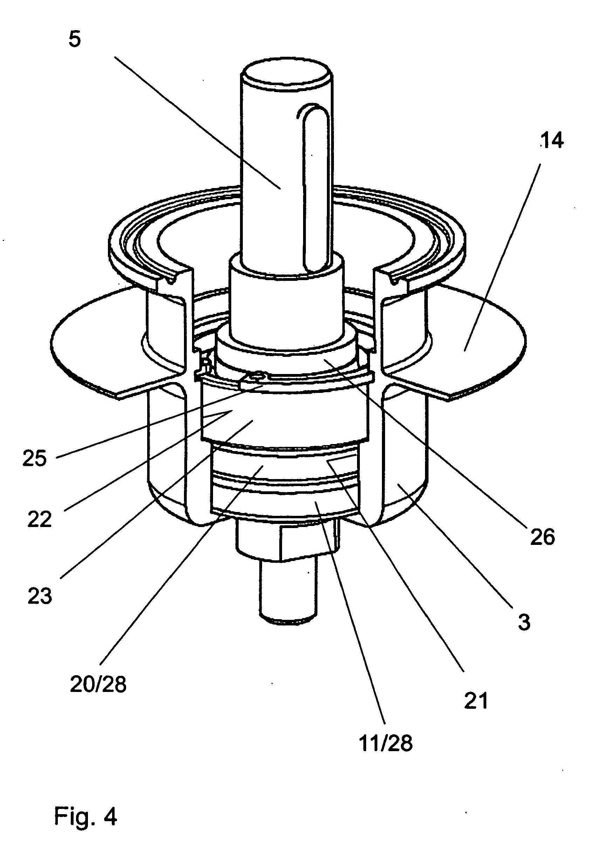

- Figur 4:: eine räumliche Darstellung eines Wellengehäuses.

Ein Bioreaktor 1 besteht im Wesentlichen aus einem Behälter 2, einem Wellengehäuse 3, einem Antrieb 4, einer Welle 5 und einem Rührer 6.

Der Behälter 2 weist eine flexible Wandung 7 auf, die in vertikaler Richtung oben zur Durchführung der Welle 5 fest mit dem Wellengehäuse 3 verbunden ist. Das Wellengehäuse 3 ist topfförmig ausgebildet und ragt mit seinem einen Boden 8 aufweisenden Unterteil in einen Innenraum 9 des Behälters 2 hinein. Der Boden 8 weist eine Durchlassöffnung 10 für die Welle 5 auf und bildet ein Stützlager für eine erste Dichtung 11, die mit ihrer Stirnfläche 12 auf den Boden 8 aufliegt. Das Wellengehäuse 3 weist eine zylinderförmige Außenwandung 13 mit einem radial umlaufenden, flachen Bund 14 auf, der mit der benachbarten, flexiblen Wandung 7 des Behälters 2 verbunden ist. Die Verbindung kann beispielsweise durch Verkleben oder Ultraschallschweißen erfolgen.

An seinem dem Boden 8 abgewandten oberen Rand weist das Wellengehäuse 3 einen Flansch 15 mit einer Ringnut 16 zur Aufnahme einer Flanschdichtung 17 auf. An den Flansch 15 ist der Antrieb 4 mit einem Flansch 18 anflanschbar. Zur Verbindung mit der Welle 5 weist der Antrieb 4 ein Kupplung 19 auf. In Längsrichtung der Welle 5 ist der ersten Dichtung 11 eine zweite Dichtung 20 benachbart angeordnet. Die beiden Dichtungen 11, 20 sind dabei in einem unteren Absatz 21 des Wellengehäuses 3 angeordnet, der in vertikaler Richtung nach unten von dem Boden 8 begrenzt wird. Dem unteren Absatz 21 ist im Wellengehäuse 3 ein in vertikaler Richtung nach oben zweiter Absatz 22 zur Aufnahme eines Kugellagers 23 vorgelagert. Den zweiten Absatz 22 benachbart weist das Wellengehäuse 3 eine Ringnut 24 auf, in der ein das Kugellager 23 fixierender Federring bzw. Sprengring 25 eingesetzt ist. Die Welle 5 weist einen umlaufenden Bund 26 auf, der als Anschlag für das auf ihr fest angeordnete Kugellager 23 dient. Über das Kugellager 23 und den Springring 25 wird die Welle 5 somit an dem Wellengehäuse 3 in ihrer Längsrichtung fixiert. Der Rührer 6 ist über seinen Schaft 27 mit der ihn antreibenden Welle 5 verbunden. Dabei kann der Schaft 27 zur Anpassung an unterschiedliche Behältergrößen mehrteilig ausgebildet sein. Die Dichtungen 11, 20 sind als ein Radialwellendichtring 28 ausgebildet. Der Radialwellendichtring 28 weist in vertikaler Richtung unten die Stirnfläche 29 auf. In radialer Richtung geht die Stirnfläche 29 in eine erste Dichtlippe 30 über, die bei hydraulischen Anwendungen eine Art dichtende Schutzlippe bildet. Der ersten Dichtlippe 30 schließt sich in vertikaler Richtung nach oben eine zweite Dichtlippe 31 an, die von einem Federring 32 dichtend gegen die Wellenoberfläche 33 der Welle 5 gedrückt wird. Die Stirnfläche 29 und die dem Wellengehäuse 3 zugewandte parallel zur Welle 5 verlaufende Außenwandung 34 des Radialwellendichtringes 28 weisen nach innen vorgelagert eine kappenförmige Verstärkung 35 auf.

## Patentansprüche

1. Bioreaktor (1) mit einem Behälter (2), einem Wellengehäuse (3) zur Durchführung einer Welle (5) durch eine Wandung (7), einem im Innenraum (9) des Behälters (2) mit der Welle (5) verbundenem Rührer (6), einem außerhalb des Behälters (2) angeordneten, mit der Welle (5) verbindbaren Antrieb (4), wobei die Welle (5) in dem Wellengehäuse (3) über mindestens ein Kugellager (23) gelagert und durch mindestens eine Dichtung (11, 20) gegenüber dem Wellengehäuse (3) abgedichtet ist, **dadurch gekennzeichnet,**
**dass** die Dichtung (11, 20) als ein Radialwellendichtring (28) mit zwei in Längsrichtung der Welle (5) hintereinander angeordneten, auf der Welle (5) trocken laufbaren Dichtlippen (30, 31) ausgebildet ist,
**dass** der Radialwellendichtring (28) zum Innenraum (9) des Behälters (2) hin eine geschlossene Stirnfläche (29) aufweist, die in radialer Richtung in eine als Schutzlippe ausgebildete Dichtlippe (30) übergeht, und dass der Radialwellendichtring (28) zum außenliegenden Antrieb (4) hin eine Dichtlippe (31) aufweist, die von einem Federring (32) dichtend gegen die Wellenöberfläche (33) gedrückt wird.

2. Bioreaktor, nach Anspruch 1,
**dadurch gekennzeichnet,**
**dass** die Wandung (7) des Behälters (2) flexibel ausgebildet ist.

3. Bioreaktor nach Anspruch 1 oder 2,
**dadurch gekennzeichnet,**
**dass** in Längsrichtung der Welle (5), der ersten Dichtung (11) benachbart, eine zweite Dichtung (20) angeordnet ist.

4. Bioreaktor nach einem der Ansprüche 1 bis 3,
**dadurch gekennzeichnet,**
**dass** die Stirnfläche (11) und die dem Wellengehäuse (5) zugewandte, parallel zur Welle (5) verlaufende Außenwandung (34) des Radialwellendichtringes (28) eine kappenförmige Verstärkung (35) aufweist.

5. Bioreaktor nach einem der Ansprüche 1 bis 4,
**dadurch gekennzeichnet,**
**dass** das Wellengehäuse (3) topfförmig ausgebildet ist und mit seinem einen Boden (8) aufweisenden Unterteil in den Innenraum (9) des Behälters (2) hineinragt, wobei der Boden (8) eine Durchlassöffnung (10) für die Welle (5) aufweist und ein Stützlager für die mindestens eine Dichtung (11) bildet, die mit ihrer Stirnfläche (12) auf dem Boden (8) aufliegt.

6. Bioreaktor nach Anspruch 5,
**dadurch gekennzeichnet,**
**dass** das Wellengehäuse (3) eine zylinderförmige Außenwandung (13) mit einem radial umlaufenden flachen Bund (14) aufweist, der mit der benachbarten flexiblen Wandung (7) des Behälters (2) verbunden ist.

7. Bioreaktor nach Anspruch 5 oder 6,
**dadurch gekennzeichnet,**
**dass** das Wellengehäuse (3) an seinem dem Boden (8) abgewandten oberen Rand einen Flansch (15) mit einer Ringnut (16) zur Aufnahme einer Flanschdichtung (17) aufweist und
**dass** an den Flansch (15) ein entsprechender Flansch (18) des Antriebs (4) anflanschbar ist.

8. Bioreaktor nach einem der Ansprüche 1 bis 7,
**dadurch gekennzeichnet,**
**dass** das dem Rührer (6) abgewandte freie Ende der Welle (5) an eine Antriebswelle (4) eines Motor des Antrieb (4) ankoppelbar ist.

9. Bioreaktor nach Anspruch 8,
**dadurch gekennzeichnet,**
**dass** das freie Ende der Welle (5) über eine Kupplung (19) an die Antriebswelle (5) ankoppelbar ist.

10. Bioreaktor nach einem der Ansprüche 1 bis 9,
**dadurch gekennzeichnet,**
**dass** die Welle (5) in ihrer Längsrichtung über den Sitz des Kugellagers (23) fixiert ist,
**dass** das Kugellager (23) in einem Absatz (21) des Wellengehäuses (3) angeordnet ist, und
**dass** das Kugellager (23) über einen in einer Ringnut (16) des Wellengehäuses (3) angeordneten Sprengring (25) in seiner Lage gesichert ist.

## Claims

1. A bioreactor (1) having a container (2), a shaft housing (3) for guiding a shaft (5) through a wall (7), a stirrer (6) connected to the shaft (5) in the interior (9) of the container (2), and a drive (4) arranged outside the container (2) and connectable to the shaft (5), said shaft (5) being mounted in the shaft housing (3) via at least one ball bearing (23) and being sealed in relation to the shaft housing (3) by at least one seal (11, 20)
**characterized in that**
the seal (11, 20) is configured as a radial shaft sealing ring (28) having two sealing lips (30, 31) that are arranged one behind the other in the longitudinal direction of the shaft (5) and are able to run on the shaft (5) without lubricant, and also **characterized in that**,
in a direction toward the interior (9) of the container (2), the radial shaft sealing ring (28) has a closed end face (29) which merges in a radial direction into a sealing lip (30) configured as a protective lip,
and further **characterized in that**,
in the direction toward the externally located drive (4), the radial shaft sealing ring (28) has a sealing lip (31) that is pressed against the shaft surface (33) by a spring ring (32) in order to produce the sealing.

2. A bioreactor according to Claim 1,
**characterized in that**
the wall (7) of the container (2) is flexible.

3. A bioreactor according to Claim 1 or 2
**characterized in that**
a second seal (20) is arranged adjacent to the first seal (11) in the longitudinal direction of the shaft (5).

4. A bioreactor according to any of the Claims 1 to 3,
**characterized in that**
the end face (11) and the outer wall (34) of the radial shaft sealing ring (28) facing toward the shaft housing (5) and extending parallel to the shaft (5) have a cap-shaped reinforcement (35).

5. A bioreactor according to any of Claims 1 to 4,
**characterized in that**
the shaft housing (3) is pot-shaped and its lower part, which has a floor (8), extends into the interior (9) of the container (2), said floor (8) having a through-opening (10) for the shaft (5) and forming a support for the at least one seal (11) that bears with its end face (12) on the floor (8).

6. A bioreactor according to Claim 5,
**characterized in that**
the shaft housing (3) has a cylindrical outer wall (13) with a radially circumferential flat collar (14) that is connected to the adjacent flexible wall (7) of the container (2).

7. A bioreactor according to Claim 5 or 6
**characterized in that**
at its upper edge facing away from the floor (8), the shaft housing (3) has a flange (15) having an annular groove (16) for receiving a flange seal (17) and also **characterized in that**
a corresponding flange (18) of the drive (4) can be flanged onto the flange (15).

8. A bioreactor according to any of Claims 1 to 7,
**characterized in that**
the free end of the shaft (5) facing away from the stirrer (6) can be coupled to a drive shaft (4) of a motor of the drive (4).

9. A bioreactor according to Claim 8,
**characterized in that**
the free end of the shaft (5) can be coupled to the drive shaft (5) via a coupling (19).

10. A bioreactor according to any of Claims 1 to 9,
**characterized in that**
in its longitudinal direction the shaft (5) is fixed via the seat of the ball bearing (23),
also **characterized in that**
the ball bearing (23) is arranged in a recess (21) of the shaft housing (3), and further **characterized in that**
the ball bearing (23) is held in position by a circlip (25) arranged in an annular groove (16) of the shaft housing (3).

## Revendications

1. Bioréacteur (1) comportant un récipient (2), un carter d'arbre (3) pour passer un arbre (5) à travers une paroi (7), un agitateur (6) relié à l'arbre (5) dans l'espace intérieur (9) du récipient (2), un entraînement (4) disposé à l'extérieur du récipient (2), pouvant être relié avec l'arbre (5), ledit arbre (5) étant monté dans le carter d'arbre (3) via au moins un roulement à billes (23) et étant étanchéifié par rapport au carter d'arbre (3) par au moins un joint (11, 20),
**caractérisé en ce que**
le joint (11, 20) est configuré en tant que joint d'arbre radial (28) présentant deux lèvres d'étanchéité (30, 31) capables de tourner à sec sur l'arbre (5), disposées l'une derrière l'autre en direction longitudinale de l'arbre (5),
et **caractérisé en ce que**,
en direction de l'espace intérieur (9) du récipient (2), le joint d'arbre radial (28) présente une extrémité fermée (29) qui fusionne en direction radiale avec une lèvre d'étanchéité (30) configurée en tant que lèvre protectrice,
et en outre **caractérisé en ce que**,
en direction de l'entraînement (4) situé à l'extérieur, le joint d'arbre radial (28) présente une lèvre d'étanchéité (31) pressée contre la surface d'arbre (33) par un joint élastique (32) afin de produire l'étanchéité.

2. Bioréacteur selon la revendication 1,
**caractérisé en ce que**
la paroi (7) du récipient (2) est flexible.

3. Bioréacteur selon la revendication 1 ou 2
**caractérisé en ce que**
un deuxième joint (20) jouxte le premier joint (11) dans la direction longitudinale de l'arbre (5).

4. Bioréacteur selon l'une quelconque des revendications 1 à 3,
**caractérisé en ce que**
l'extrémité (11) et la paroi externe (34) du joint d'arbre radial (28) opposé au carter d'arbre (5) et s'étendant parallèlement à l'arbre (5) comportent un renfort en forme de chapeau (35).

5. Bioréacteur selon l'une quelconque des revendications 1 à 4,
**caractérisé en ce que**
le carter d'arbre (3) est en forme de pot et que sa partie inférieure, qui comporte un plancher (8), s'étend dans l'espace intérieur (9) du récipient (2), ledit plancher (8) ayant une ouverture de passage (10) pour l'arbre (5) et formant un appui pour le minimum d'un joint (11) dont l'extrémité (12) repose sur le plancher (8).

6. Bioréacteur selon la revendication 5,
**caractérisé en ce que**
le carter d'arbre (3) comporte une paroi externe cylindrique (13) avec un collet plat radialement circonférentiel (14) relié à la paroi flexible attenante (7) du récipient (2).

7. Bioréacteur selon la revendication 5 ou 6
**caractérisé en ce que**
sur son bord supérieur opposé au plancher (8), le carter d'arbre (3) comporte une bride (15) ayant une rainure annulaire (16) pour recevoir un joint de bride (17) et **en ce que**,
une bride correspondante (18) de l'entraînement (4) peut être fixée à la bride (15).

8. Bioréacteur selon l'une quelconque des revendications 1 à 7,
**caractérisé en ce que**
l'extrémité libre de l'arbre (5) opposée à l'agitateur (6) peut être raccordée à un arbre d'entraînement (4) d'un moteur de l'entraînement (4).

9. Bioréacteur selon la revendication 8,
**caractérisé en ce que**
l'extrémité libre de l'arbre (5) peut être raccordée à un arbre d'entraînement (5) via un raccord (19).

10. Bioréacteur selon l'une quelconque des revendications 1 à 9,
**caractérisé en ce que**
dans sa direction longitudinale, l'arbre (5) est fixé via le siège du roulement à billes (23),
et **en ce que**,
le roulement à billes (23) est disposé dans une rainure (21) du carter d'arbre (3), en caractérisé outre **en ce que**,
le roulement à billes (23) est maintenu en place par un circlip (25) disposé dans une rainure annulaire (16) du carter d'arbre (3).
